(19) Europäisches Patentamt

European Patent Office

Office européen des brevets

(11) Publication number: **0 230 700**
**B1**

(12) **EUROPEAN PATENT SPECIFICATION**

(45) Date of publication of the patent specification:
20.09.89

(51) Int. Cl.⁴: **C07C 11/167**, C07C 7/12

(21) Application number: 86300353.9

(22) Date of filing: 20.01.86

(54) Separation of 1,3-butadiene.

(43) Date of publication of application:
05.08.87 Bulletin 87/32

(45) Publication of the grant of the patent:
20.09.89 Bulletin 89/38

(84) Designated Contracting States:
AT BE DE FR GB IT NL SE

(56) References cited:
US-A- 2 985 589
US-A- 3 596 436
US-A- 3 992 471
US-A- 4 402 832

CHEMICAL ABSTRACTS, vol. 66, no. 18, 1st May 1967,
page 7495, abstract no. 79923x, Columbus, Ohio, US;
Z.E. ALIEV et al.: "Increase of efficiency of carbon
adsorbents for the separation of highly concentrated
butadiene from a butylene-butadiene fraction" &
METALL (BERLIN) 1985, 39(8), 715-2036(5), 225-31 000

(73) Proprietor: UOP, 25 East Algonquin Road, Des Plaines
Illinois 60017-5017(US)

(72) Inventor: Kulprathipanja, Santi, 3920 Winston Drive,
Hoffman Estates Illinois 60172(US)

(74) Representative: Brock, Peter William,
URQUHART-DYKES & LORD 91 Wimpole Street, London
W1M 8AH(GB)

## Description

The present invention relates to a process for separating 1, 3-butadiene from a feed mixture comprising 1, 3-butadiene and at least one other C$_4$ hydrocarbon by adsorption and desorption.

It is well known in the separation art that certain crystalline aluminosilicates can be used to separate one hydrocarbon type from another hydrocarbon type. The separation of normal paraffins from branched chain paraffins, for example, can be accomplished by using a type A zeolite which has pore openings from 3 to 5 Angstroms. Such a separation process is disclosed in US-A-2 985 589 and 3 201 491. These adsorbents allow a separation based on the physical size differences in the molecules by allowing the smaller or normal hydrocarbons to be passed into the cavities within the zeolitic adsorbent, while excluding the larger or branched chain molecules.

In addition to being used in processes for separating hydrocarbon types, adsorbents comprising type X or Y zeolites have also been employed in processes to separate individual hydrocarbon isomers. In the processes described, for example, in US-A 3 626 020, -3 663 638, -3 665 046, -3 668 266, -3 686 343, -3 700 744, -3 734 974, -3 894 109, -3 997 620 and -B 4 26 274 particular zeolitic adsorbents are used to separate the para isomer of bi-alkyl substituted monocyclic aromatics from the other isomers, particularly para-xylene from other xylene isomers.

There is also separation art that deals specifically with the separation of 1, 3-butadiene from other C$_4$ hydrocarbons, particularly monoolefins. US-A 3 311 671 and -3 992 471 teach the use of alkali metal-aluminium silicates on zeolites in effecting that separation. More specifically, US-A 3 992 471 discloses a process in which butadiene is selectively adsorbed from a C$_4$ diene/monoene mixture on type X zeolite with sodium or potassium at cationic sites, the butadiene being desorbed by means of an aromatic hydrocarbon, optionally a mixture of aromatic hydrocarbon with paraffins or naphthenes. US-A 3 596 436 discloses the use of activated charcoal to separate diolefins from monoolefins, but requires that the entire process be carried out in the vapor phase and provides for the regeneration of the solid adsorbent (removal of diolefins) with superheated steam.

Aliev et al disclose in Azerb. Khim. Zh. 1966(4), 56–61 (Chemical Abstracts Vol 66 (18), 1967, page 7495, Abstract 79923x) that a carbon guard bed can be used to remove impurities from a butylene/butadiene fraction, the carbon being subsequently regenerated by sulfuric or acetic acid.

The present inventions seek to provide an alternative process for the separation of butadiene from other C$_4$ hydrocarbons, preferably one having advantages as compared with prior proposals.

According to the present invention, a process for separating 1, 3-butadiene from a feed mixture comprising 1, 3-butadiene and at least one other C$_4$ hydrocarbon comprises contacting the feed mixture in liquid phase with an adsorbent comprising activated or molecular sieve carbon, whereby the 1, 3-butadiene is selectively adsorbed, removing the unadsorbed portion of the feed from the adsorbent, and recovering the 1, 3-butadiene by desorption with a liquid desorbent material comprising an n-olefin of 3 or 5 to 10 carbon atoms, having a boiling point at least 5°C different from that of the feed mixture.

Thus, 1, 3-butadiene is the extract component in the process of the invention and the one or more other C$_4$ hydrocarbons constitute the raffinate component, although the compositions of the raffinate stream and extract stream through which they are removed from the adsorbent can vary from essentially 100% desorbent material (i.e. material capable of desorbing the extract component) to essentially 100% raffinate or extract components. Moreover, although it is possible by the process of this invention to produce high-purity extract product or raffinate product at high recoveries, it will be appreciated that an extract component is never completely adsorbed by the adsorbent, nor is a raffinate component completely non-adsorbed by the adsorbent. Therefore, small amounts of a raffinate component can appear in the extract stream and likewise, small amounts of an extract component can appear in the raffinate stream. The extract and raffinate streams then are further distinguished from each other and from the feed mixture by the ratio of the concentrations of an extract component and a specific raffinate component, both appearing in the particular stream. For example, the ratio of concentration of the more selectively adsorbed 1,3-butadiene to the concentration of less selectively adsorbed other C$_4$ hydrocarbons will be highest in the extract stream, next highest in the feed mixture, and lowest in the raffinate stream. Likewise, the ratio of the less selectively adsorbed other C$_4$ hydrocarbons to the more selectively adsorbed 1,3-butadiene will be highest in the raffinate stream, next highest in the feed mixture, and the lowest in the extract stream. When the extract stream and the raffinate stream contain desorbent materials, at least a portion of the extract stream and preferably at least a portion of the raffinate stream from the adsorbent will be passed to separation means, typically fractionators, where at least a portion of desorbent material will be separated at separation conditions to produce an extract product and a raffinate product, namely products produced by the process containing, respectively, an extract component and a raffinate component in higher concentrations than those found in the respective extract stream and the raffinate stream.

1,3-Butadiene, industrially the most important diolefin, is used to produce polymer components used, for example, in synthetic rubber and is also used as a chemical intermediate for a great variety of compounds.

Butadiene is synthesized commercially by four main methods: (1) by catalytic dehydrogenation of concentrated n-butylenes; (2) by catalytic dehydrogenation of n-butane; (3) as a by-product, in rather low yield, from severe high-temperature cracking of liquid hydrocarbons for production of unsaturates; and (4) from ethyl alcohol by a combination of catalytic dehydrogenation and dehydration. The first two methods are most frequently used.

All of the conversion processes yield products in which 1,3-butadiene is mixed with other closely boiling hydrocarbons. For example, when concentrated 2-butene and 1-butene are catalytically dehydrogenated to produce 1,3-butadiene the stabilized effluent from this operation contains, in addition to 1,3-butadiene, unreacted isomeric n-butenes, some n-butane, isobutane, isobutylene, appreciable concentration of $C_3$ components, and small concentrations of components heavier than $C_4$ hydrocarbons.

Table 1 below lists the hydrocarbons frequently found in crude butadiene fractions from such sources and is indicative of the composition of the feed mixture that might be expected for use in the process of the present invention. The relative amounts of these hydrocarbons present in crude butadiene vary considerably, depending upon the type of hydrocarbon conversion process employed. Other $C_4$ unsaturates, primarily monoolefins, are always present in major amounts. Non-conjugated diolefins and acetylenes are minor constituents, but they generally increase with increasing temperature during hydrocarbon conversion. For the most part, however, they are highly objectionable contaminants in purified butadiene and hence their concentrations in the latter must be carefully controlled.

Table 1

| Composition of crude butadiene fractions | | |
|---|---|---|
| | BP. °C | Vol.% |
| $C_3$ Hydrocarbons | | 0.9 |
| Isobutylene | −6.9 | 27.7 |
| 1-Butene | −6.3 | 17.2 |
| 1,3-Butadiene | −4.4 | 39.1 |
| n-Butane | −0.5 | 4.1 |
| t-2-Butene | +0.9 | 6.0 |
| c-2-Butene | +3.7 | 4.5 |
| $C_4$ Acetylenes | +5.1 | 0.2 |
| 1,2-Butadiene | +10.9 | <0.1 |
| $C_5$ Hydrocarbons | | 0.1 |

To separate the 1,3-butadiene from a feed mixture in accordance with the present invention, the mixture is contacted with the adsorbent and the 1,3-butadiene is more selectively adsorbed and retained by the adsorbent while the other components of the feed mixture are relatively unadsorbed and are removed from the interstitial void spaces between the particles of adsorbent and the surface of the adsorbent. The adsorbent containing the more selectively adsorbed 1.3-butadiene is referred to as a "rich" adsorbent. The 1,3-butadiene is then recovered from the rich adsorbent by contacting the rich adsorbent with a desorbent material.

Desorbent materials should be substances which are easily separable from the feed mixture that is passed into the process. After desorbing the 1, 3-butadiene, both desorbent material and the 1, 3-butadiene are typically removed in admixture from the adsorbent. Likewise, one or more of the other $C_4$ hydrocarbons are typically withdrawn from the adsorbent in admixture with desorbent material and, without a method of separating at least a portion of desorbent material, such as distillation, neither the purity of the extract product nor the purity of the raffinate product would be very high.

Accordingly, the desorbent material should have a substantially different average boiling point from that of the feed mixture, to allow separation of desorbent material by simple fractionation thereby permitting reuse of desorbent material in the process. The term "substantially different" means that the difference between the average boiling points between the desorbent material and the feed mixture is at least 5°C. The boiling range of the desorbent material may be higher or lower than that of the feed mixture.

In the process of this invention, the effective desorbent materials are $C_3$ or $C_5$ to $C_{10}$ n-olefins. The ability to operate in the liquid phase, in contradistinction to the aforemented US-A 3 596 436, is a distinct advantage in view of the energy savings and relative simplicity of operation.

An important property of the adsorbent is the degree, or lack thereof, to which it chemically reacts with or causes chemical change to the feed and desorbent components. US-A-3,311,671 (Baker) and 3,992,471 (Priegnitz), for example, teach the use of zeolitic materials as adsorbents. Such materials, however, are known to react with hydrocarbons, particularly olefins. In contradistinction to such chemically active adsorbents, the carbon adsorbents proposed in the present invention are chemically inert to the components of the feed mixture and the desorbent material.

In order to test various adsorbents and desorbent material with a particular feed mixture to measure the adsorbent characteristics of adsorptive capacity and selectivity and exchange rate, a dynamic testing apparatus is employed. The apparatus consists of an adsorbent chamber of approximately 70 cc volume having inlet and outlet portions at opposite ends of the chamber. The chamber is contained within a temperature control means and, in addition, pressure control equipment is used to operate the chamber at a constant predetermined pressure. Chromatographic analysis equipment can be attached to the outlet line of the chamber and used to analyze "on-stream" the effluent stream leaving the adsorbent chamber.

A pulse test, performed using this apparatus and the following general procedure, is used to determine selectivities and other data for various adsorbent systems. The adsorbent is filled to equilibrium with a particular desorbent by passing the desorbent material through the adsorbent chamber. At a convenient time, a pulse of feed containing known concentrations of a non-adsorbed paraffinic tracer (n-nonane for instance) and of the particular feed mixture of $C_4$ hydrocarbons diluted in desorbent is injected for a duration of several minutes. Desorbent flow is resumed, and the tracer and the constituents of the feed mixture are eluted as in a liquid-solid chromatographic operation. The effluent can be analyzed by on-stream chromatographic equipment and traces of the envelopes of corresponding component peaks developed. Alternately, effluent samples can be collected periodically and later analyzed separately by gas chromatography.

From information derived from the chromatographic traces, adsorbent performance can be rated in terms of capacity index for an extract component, selectivity for one $C_4$ hydrocarbon with respect to the other, and the rate of desorption of an extract component by the desorbent. The capacity index may be characterized by the distance between the center of the peak envelope of the selectively adsorbed component and the peak envelope of the tracer component or some other known reference point. It is expressed in terms of the volume in cubic centimeters of desorbent pumped during this time interval. Selectivity, (B), for an extract component with respect to a raffinate component may be characterized by the ratio of the distance between the center of an extract component peak envelope and the tracer peak envelope (or other reference point) to the corresponding distance between the center of a raffinate component peak envelope and the tracer peak envelope. The rate of exchange of an extract component with the desorbent can generally be characterized by the width of the peak envelopes at half intensity. The narrower the peak width the faster the desorption rate. The desorption rate can also be characterized by the distance between the center of the tracer peak envelope and the disappearance of an extract component which has just been desorbed. This distance is again the volume of desorbent pumped during this time interval.

The adsorbent to be used in the process of this invention comprises what is known as activated carbon or molecular sieve carbon. Activated carbon is a common, commercially available material, such as Calgon Corporation's "Type PCB" granular carbon, or Union Carbide Corporation's material having the trademark "PURASIV". Type PCB as described in Calgon's brochure No. 23-108a, dated August 1978, is an activated carbon having a large portion of micropore volume in pores of 15 to 20 Angstrom units in diameter permeated by a system of macropores larger than 1000 Angstroms in diameter. PURASIV, as described in Union Carbide's brochure F-4866815M, is a beaded activated carbon made from molten petroleum pitch shaped into spherical particles and subsequently carbonized and activated.

The term "molecular sieve carbon" as used herein is not intended to necessarily distinguish from those materials referred to as "activated carbon" but to ensure that no material effective for use in the present invention is excluded. There is considerable overlap between the two terms in question and probably in most instances, for purposes of the present invention, the terms are interchangeable. The particular molecular sieve carbons known to be effective for use in the present invention are those having an average pore size in excess of 5 Angstrom units.

The adsorbent may be in the form of particles such as extrudates, aggregates, tablets, macrospheres or granules having a desired particle range, preferably from 16 to 60 mesh (Standard U.S. Mesh) which corresponds to a nominal aperture of 1.19 mm to 0.25 mm. Less water content in the adsorbent is advantageous from the standpoint of less water contamination of the product.

The adsorbent may be employed in the form of a dense fixed bed which is alternatively contacted with a feed mixture and a desorbent material in which case the process will be only semi-continuous. In another embodiment, a set of two or more static beds of adsorbent may be employed with appropriate valving so that a feed mixture can be passed through one or more adsorbent beds of a set while a desorbent material can be passed through one or more of the other beds in a set. The flow of a feed mixture and a desorbent material may be either up or down through an adsorbent in such beds. Any of the conventional apparatus employed in static bed fluid-solid contacting may be used.

Moving bed or simulated moving bed flow systems, however, have a much greater separation efficiency than fixed bed systems and are therefore preferred. In the moving bed or simulated moving bed processes, the retention and displacement operations are continuously taking place which allows both continuous production of an extract and a raffinate stream and the continual use of feed and displacement fluid streams. One preferred embodiment of this process utilizes what is known in the art as the simulated moving bed countercurrent flow system. In such a system, it is the progressive movement of multiple liquid access points down a molecular sieve chamber that simulates the upward movement of molecular sieve contained in the chamber. Reference can also be made to US-A 2 985 589, in which the operating principles and sequence of such a flow system are described, and to a paper entitled, "Continuous Adsorptive Processing -- A New Separation Technique" by D.B. Broughton presented at the 34th Annual Meeting of the Society of Chemical Engineers at Tokyo, Japan on April 2, 1969, for further explanation of the simulated moving bed countercurrent process flow scheme.

Another embodiment of a simulated moving bed flow system suitable for use in the process of the present invention is the co-current high efficiency simulated moving bed process disclosed in US-A 4 402 832.

It is contemplated that at least a portion of the extract output stream will pass into a separation means wherein at least a portion of the desorbent material can be separated at separating conditions to produce an extract product containing a reduced concentration of desorbent material. Preferably, but not necessary to the operation of the process, at least a portion of the raffinate output stream will also be passed to a separation means wherein at least a portion of the desorbent material can be separated at separating conditions to produce a desorbent stream which can be reused in the process and a raffinate product containing a reduced concentration of desorbent material. Typically the concentration of desorbent material in the extract product and the raffinate product will be less than 5 vol. % and more preferably less than 1 vol. %. The separation means will typically be a fractionation column, the design and operation of which is well known to the separation art.

Although both liquid and vapor phase operations can be used in many adsorptive separation processes, liquid-phase operation is required for this process because of the lower temperature and energy requirements and because of the higher yields of an extract product that can be obtained with liquid-phase operation over those obtained with vapor-phase operation. Adsorption conditions will include a temperature range of from 20°C to 250°C, with 100°C to about 200°C being more preferred, and a pressure sufficient to maintain liquid phase. Desorption conditions will include the same range of temperatures and pressure as used for adsorption conditions.

The size of the units which can utilize the process of this invention can vary anywhere from those of pilot-plant scale (see for example US-A 3 706 812) to those of commercial scale and can range in flow rates from as little as a few cc an hour up to many thousands of gallons per hour.

The following examples are presented for illustration purposes and more specifically are presented to illustrate the selectivity relationships that make the process of the invention possible.

## EXAMPLE 1

In this experiment, three pulse tests were performed to evaluate the ability of the present invention to separate 1,3-butadiene from other $C_4$ hydrocarbons using three different carbon adsorbents.

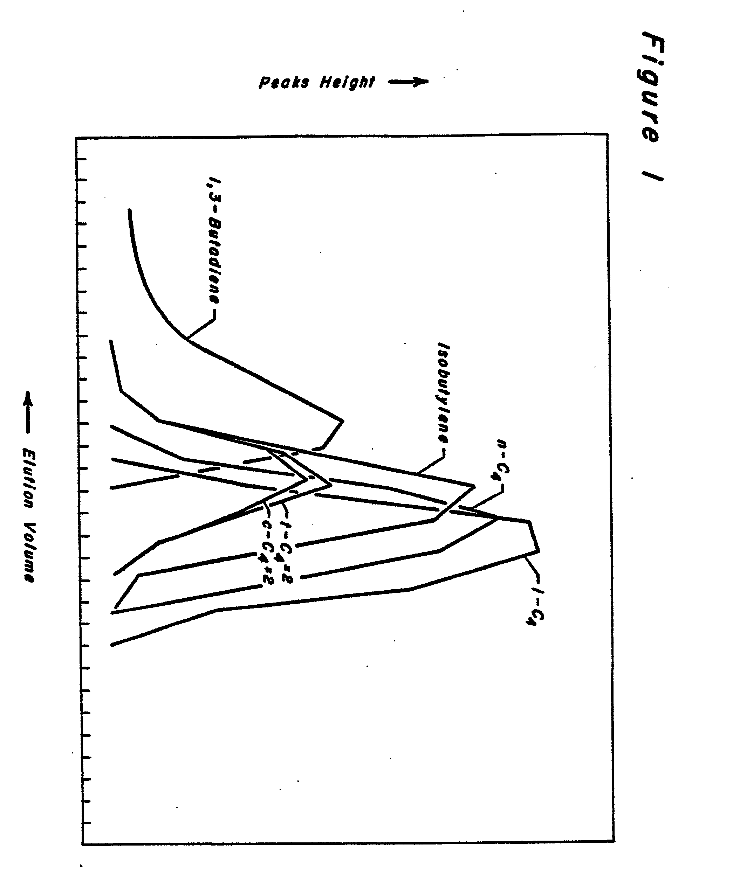

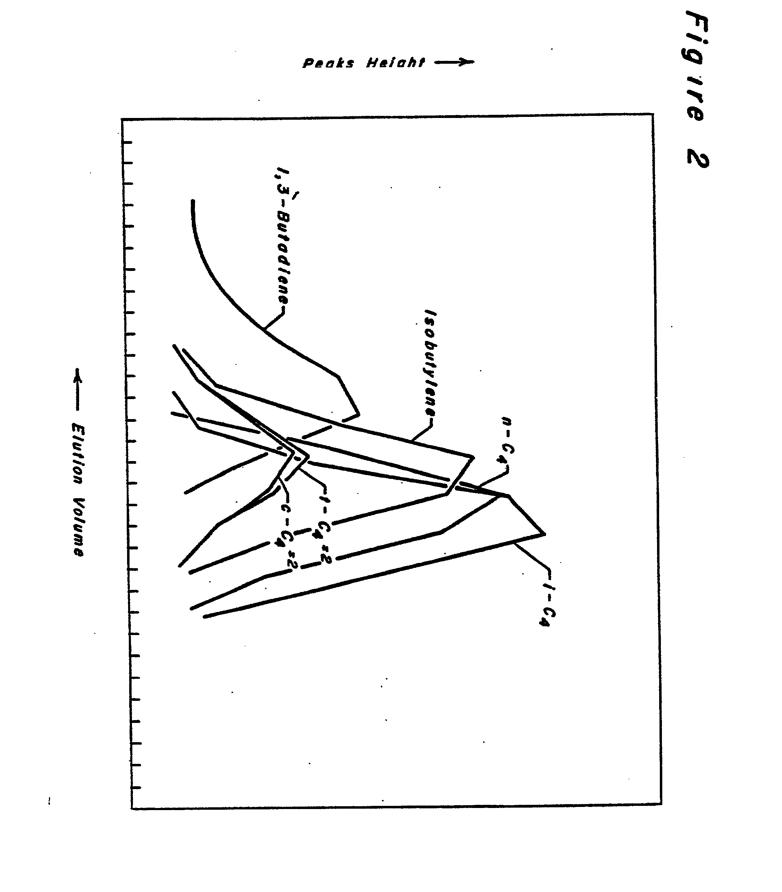

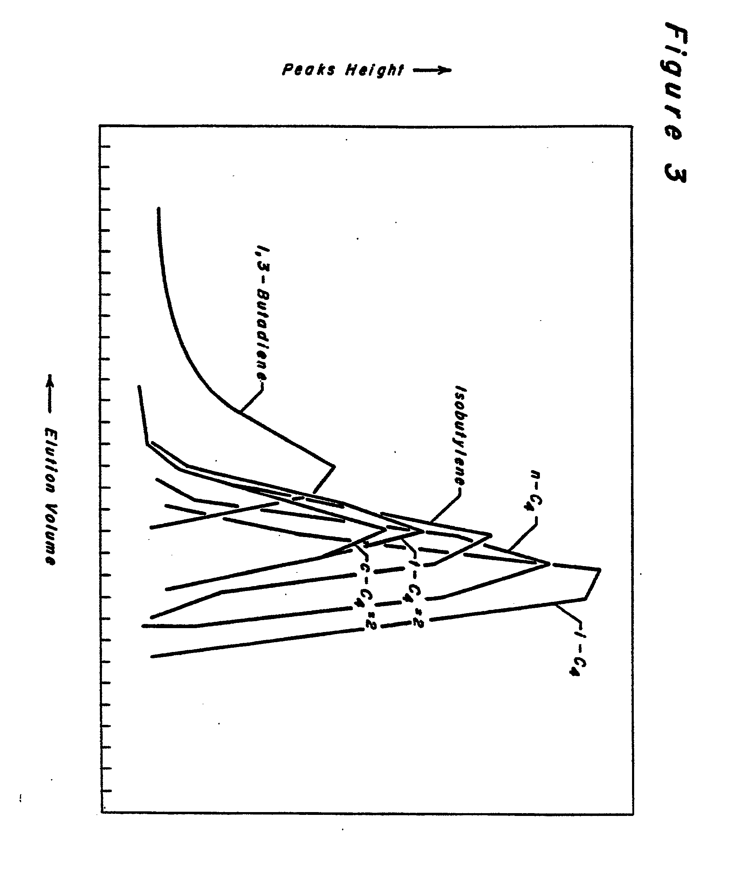

The testing apparatus was the above described pulse test apparatus. For each pulse test, the column was maintained at a temperature of 65°C and a pressure sufficient to maintain liquid-phase operations. Gas chromatographic analysis equipment was attached to the column effluent stream in order to determine the composition of the effluent material at given time intervals. The feed mixture employed for each test contained about 5 vol. % each of isobutane, normal butane, isobutylene, trans-butene-2, cis-butene-2 and 1,3-butadiene and 70 vol. % desorbent material. The desorbent material was hexene-1. The operations taking place for each test were as follows. The desorbent material was run continuously at a nominal liquid hourly space velocity (LHSV) of 1.0 which amounted to about 1.17 cc per minute feed rate of desorbent. At some convenient time interval the desorbent was stopped and the feed mixture was run for a 10 minute interval at a rate of 1.0 cc per minute. The desorbent stream was then resumed at 1 LHSV and continued to pass into the adsorbent column until all of the feed components had been eluted from the column as determined by observing the chromatograph generated by the effluent material leaving the adsorption column. The sequence of operations usually takes about an hour. The 10 minute pulse of feed and subsequent desorption may be repeated in sequence as often as is desired. The chromatograph tracings obtained are as shown in the attached Figures 1, 2 and 3. Selectivities derived from the traces are given in Table 2, the reference curve being isobutane which is presumed to be totally non-adsorbed.

| Table 2 | | | |
|---|---|---|---|
| Figure No. | 1 | 2 | 3 |
| Adsorbent | Calgon PCB | Purasiv | Molecular Sieve Carbon |
| 1,3-butadiene/n-butane | 6.26 | 6.17 | 5.75 |
| 1,3-butadiene/isobutylene | 2.59 | 2.54 | 3.74 |
| 1,3-butadiene/trans-butene | 2.05 | 2.12 | 2.13 |
| 1,3-butadiene/cis-butene | 2.06 | 2.18 | 2.17 |

The tracings of the figures for all three tests show a clear and distinct separation of 1,3-butadiene from the other feed components. The 1,3-butadiene is the last component to elute from each pulse test which is indicative of being the most selectively retained component with all three adsorbents. The high selectivities given in Table 2 for 1,3-butadiene derived from the curves provides a quantitative measure of the high degree of relative retention of the 1,3-butadiene.

**Claims**

1. A process for separating 1, 3-butadiene from a feed mixture comprising 1, 3-butadiene and at least one other C₄ hydrocarbon, which process comprises contacting the feed mixture in liquid phase at adsorption conditions with an adsorbent which selectively adsorbs said 1, 3-butadiene, removing the unadsorbed portion of said feed from said adsorbent, and recovering said 1, 3-butadiene by desorption at desorption conditions with a liquid desorbent material comprising a hydrocarbon, said feed mixture and said desorbent material having boiling points of at least 5°C difference, characterised in that the adsorbent comprises activated or molecular sieve carbon, and in that the desorbent material comprises an n-olefin of 3 or of 5 to 10 carbon atoms.

2. A process according to claim 1, characterised in that the adsorption and desorption conditions include a temperature within the range of from 20°C to 250°C and a pressure sufficient to maintain liquid phase.

3. A process according to claim 1 or 2 characterised in that the process is effected with a simulated moving bed flow system.

4. A process according to claim 3, characterised in that the simulated moving bed flow system is of the countercurrent type.

5. A process according to claim 3, characterised in that the simulated moving bed flow system is of the co-current high efficiency type.

**Patentansprüche**

1. Verfahren zur Abtrennung von l-Butadien von einem Beschickungsgemisch enthaltend 1,3-Butadien und wenigstens einen anderen C₄-Kohlenwasserstoff, indem man das Beschickungsgemisch in flüssiger Phase mit einem Adsorptionsmittel, das selektiv das genannte, 1,3-Butadien adsorbiert, in Berührung bringt, den nicht adsorbierten Anteil der Beschickung aus dem Adsorptionsmittel entfernt und das 1,3-Butadien durch Desorption unter Desorptionsbedingungen mit einem flüssigen Desorptionsmaierial, bestehend aus einem Kohlenwasserstoff, gewinnt, wobei das Beschickungsgemisch und das Desorptionsmaterial Siedepunkte, die um wenigsten 5°C differieren, besitzen, dadurch gekennzeichnet, daß das Absorptionsmittel aus Aktivkohle oder Molekularsiebkohle besteht und daß das Desorptionsmaterial ein n-Olefin mit 3 oder 5 bis 10 Kohlenstoffatomen ist.

2. Verfahren nach Anspruch 1, dadurch gekennzeichnet, daß die Apsorptions- und Desorptionsbedingungen eine Temperatur im Bereich von 20 bis 250°C und einen Druck, der ausreicht, die flüssige Phase aufrecht zu erhalten, umfassen.

3. Verfahren nach Anspruch 1 oder 2, dadurch gekennzeichnet, daß das Verfahren in einem simulierten Bewegtbett-Fließsystem durchgeführt wird.

4. Verfahren nach Anspruch 3, dadurch gekennzeichnet, daß das simulierte Bewegtbett-Fließsystem ein Gegenstromsystem ist.

5. Verfahren nach Anspruch 3, dadurch gekennzeichnet, daß das Bewegtbett-Fließsystem ein hochwirksames Gleichstromsystem ist.

**Revendications**

1. Procédé pour la séparation du butadiène-1,3 d'avec un mélange de charge comprenant du butadiène-1,3 et au moins un autre hydrocarbure en C₄, lequel procédé comprend la mise en contact du mélange de charge, en phase liquide, dans des conditions d'adsorption, avec un adsorbant qui adsorbe sélectivement ledit butadiène-1,3, l'élimination de la partie non adsorbée deladite charge hors dudit adsorbant, et la récupération dudit butadiène-1,3 par désorption dans des conditions de désorption, avec une substance désorbante liquide comprenant un hydrocarbure, ledit mélange de charge et ladite substance désorbante ayant des points d'ébullition distincts d'au moins 5°C, caractérisé en ce que l'adsorbant comprend du charbon actif ou du carbone de type tamis moléculaire, et en ce que la substance désorbante comprend une n-oléfine ayant 3 ou de 5 à 10 atomes de carbone.

2. Procédé selon la revendication 1, caractérisé en ce que les conditions d'adsorption et de désorption comprennent une température dans la plage de 20 à 250°C et une pression suffisante pour le maintien d'une phase liquide.

3. Procédé selon la revendication 1 ou 2, caractérisé en ce que le procédé est mis en œuvre avec un système a'écoulement à lit mobile simulé.

4. Procédé selon la revendication 3, caractérisé en ce que le système d'écoulement à lit mobile simulé est du type à contre-courant.

5. Procédé selon la revendication 3, caractérisé en ce que le système d'écoulement à lit mobile simulé est du type cocourant à haut rendement.

Figure I

EP 0 230 700 B1

Peaks Height →

Isobutylene

n-C₄

l-C₄

l-C₄=2

c-C₄=2

1,3-Butadiene

← Elution Volume

Figure 2

Figure 3

EP 0 230 700 B1

Peaks Height →

1 - C$_4$

n - C$_4$

Isobutylene

i - C$_4$ =2

c - C$_4$ =2

1,3 - Butadiene

← Elution Volume